# EUROPEAN PATENT SPECIFICATION

(11) **EP 0 525 046 B1**
(45) Date of publication and mention of the grant of the patent: **27.08.1997**
(21) Application number: 91908086.1
(22) Date of filing: 25.03.1991
(51) Int. Cl.: G01N 33/543, G01N 33/558, B01L 3/00, G01N 31/22

(54) **SOLID PHASE IMMUNOASSAY DEVICE AND METHOD OF MAKING SAME**
FESTPHASENIMMUNOASSAY-VORRICHTUNG UND VERFAHREN ZU SEINER HERSTELLUNG
DISPOSITIF D'ANALYSE IMMUNOLOGIQUE A PHASE SOLIDE ET METHODE DE FABRICATION DE CELUI-CI

(30) Priority: 27.03.1990 US 500005
(43) Date of publication of application: 03.02.1993
(73) Proprietor: PACIFIC BIOTECH INC., San Diego California 92121 (US)
(72) Inventor: FAN, Eugene, La Jolla, CA 92037 (US); TZENG, Sinfu, San Francisco, CA 94132 (US); CHEN, Fon-Chiu, Mia, Ramona, CA 92065 (US); HUANG, Ching, Chula Vista, CA 92011 (US); WANG, Dou-Mei, Encinitas, CA 92024 (US); POPEJOY, Theresa, Poway, CA 92064 (US)
(74) Representative: Maury, Richard Philip
(86) International application number: US9101974
(87) International publication number: WO9114942

(56) References cited:
- EP-A- 0 306 336
- EP-A- 0 323 605
- GB-A- 2 204 398
- US-A- 4 017 261
- US-A- 4 774 192
- US-A- 4 855 240
- US-A- 4 857 453
- US-A- 4 912 034
- US-A- 4 943 522
- US-A- 4 960 691

## Description

A number of devices of varying design formats presently co-exist on the market. In addition to devices requiring a multiplicity of tubes, vials and support structures, there are now various immunoassay devices encased in rigid containers or cases of varying designs, shapes and colours. For the individual manufacturer who produces a variety of devices, variations in container formats increase production costs, largely because there is no uniformity of manufacturing or assembly techniques, components differ, and labelling of each type of device must necessarily change as well.

EP-A-0296724 and GB-A-2204398 disclose immunoassay devices, each comprising a housing with a plurality of apertures in its top, wicking material inside, immunoassay reagents located on the wicking material, and absorbent material in fluid contact with the wicking material which avoids reverse flow onto the wicking material.

The present invention is in response to an express need for an immunoassay device with universal applicability, which further promotes the goals of inventory reduction and simplified, less costly manufacture.

The invention is defined in Claim 1.

In a preferred embodiment, the wicking material inside the housing extends between at least two of the apertures, wherein a first aperture is adapted to receive a sample and deposit it on a first portion of the wicking material, and a second aperture is adapted to permit viewing of the results of a completed assay on a second portion of the wicking material.

In yet another embodiment, the wicking material also extends to a third aperture, which aperture is adapted to permit application of reagents to the wicking material before application of the thin web to the housing, which third aperture is covered by the thin web. In another preferred embodiment, the wicking material also extends to a fourth aperture, the fourth aperture adapted to permit viewing of a signal on the wicking material indicative of completion of the assay. In another variation, the wicking material is in fluid flow contact with an absorbent material adjacent to the fourth aperture.

In another variation of the invention, the top of the housing further has a well formed therein for holding a container for liquid used in the assay. In another embodiment, the top of the container further has an opening therein for receiving a desiccant material, which opening is covered by the thin web. In a particularly preferred embodiment, the thin web comprises paper or plastic.

In another embodiment of the present invention, the wicking material inside the housing extends between the least three of the apertures (wherein a first such aperture is adapted to receive a sample and deposit it on a first portion of the wicking material, a second such aperture is adapted to permit viewing of the results of a completed assay on a second portion of the wicking material, and a third such aperture is located between the first and second apertures), reagents used in the assay are located on the wicking material beneath the third aperture, a well is formed in the housing adapted to hold a container of liquid used in the assay, and a thin web of opaque material is applied over the top of the housing covering one or more of the well and the third aperture.

In another embodiment, the device further contains a desiccant material in the housing with an opening provided in the top of the housing over the desiccant, wherein the thin web covers the opening. In yet another embodiment, an absobent material is in fluid contact with the wicking material, in such manner that sample is applied to the absorbent material at the first aperture prior to contacting the wicking material. In another variation, the device contains an absorbent material in fluid contact with the wicking material, in such manner that reverse flow of tracer or label, such as coloured particles (e.g. latex), back onto the wicking material is prevented. In yet another embodiment, the absorbent material further prevents the reverse flow of sample or sample components back onto the wicking material.

A method for constructing immunoassay devices, particularly those of the present invention, comprises the steps of providing a housing having an inside, an outside, and a top having a plurality of apertures communicating with the inside of the housing, inserting a web of wicking material inside the housing extending between the first, second, and third the apertures, so that the web is accessible through the first, second and third apertures and the third aperture is located between the first and second apertures, applying a first reagent to the web through the second aperture, which first reagent is bound to the web, applying a second reagent to the web through the third aperture, which second reagent is mobile when liquid is added to the first aperture, and is adapted to co-operate with an analyte in a sample and the first reagent to create a signal indicative of the results of the assay, which signal can be read through the third window, and thereafter applying a thin web of opaque material over the top of the housing over the third aperture but not over the first and second apertures, to form a first immunoassay device. The method for constructing immunoassay devices may further comprise the step of inserting an absorbent material into the housing before applying the top of the housing.

The method for constructing immunoassay devices may utilise a device wherein the top of the housing has an opening therethrough for receiving a desiccant, and may further comprise the steps of inserting a desiccant into the housing through the opening before applying the thin web, and applying the thin web over the opening.

Yet another method for constructing immunoassay devices includes the step of repeating the aforementioned steps, except that in the step of applying the thin web, at least one aperture that was covered by the thin web in the first immunoassay is not covered, to form a second immunoassay device for performing an immunoassay different from that adapted to be performed by the first immunoassay device.

### BRIEF DESCRIPTION OF THE DRAWINGS

FIG. 1 is a perspective view of a preferred embodiment of the device of the present invention.

FIG. 2 is a perspective view of the device of the preferred embodiment having an opaque label covering some of the openings shown in Fig. 1.

FIG. 3 is a perspective view of the device of the preferred embodiment having an opaque label covering some of the openings shown in Fig. 1

### DETAILED DESCRIPTION

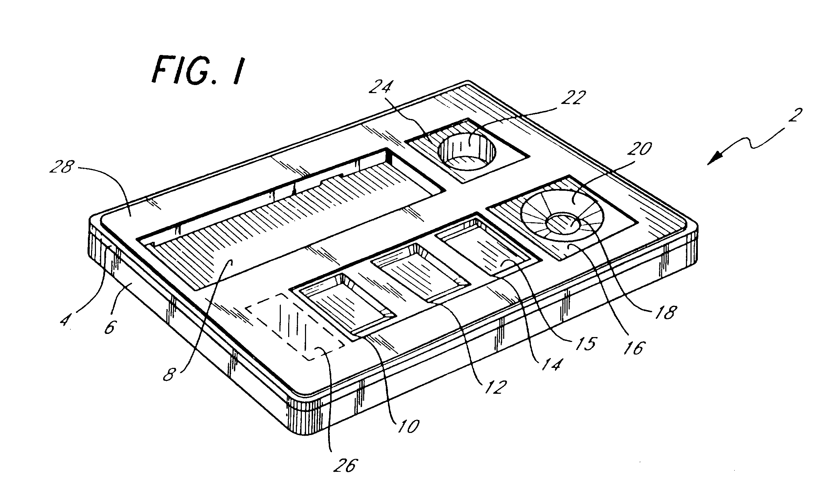

Referring to FIG. 1, there is shown an immunoassay device 2, which is generally comprised of an upper "card" member 4 and lower "card" member 6. The upper and lower card members 4, 6 are adapted to mate together to form a housing. The upper card member 4 may be provided with at least six apertures, including a tracer loading port 14, sample loading port 16, and test view ports 10 and 12. In addition, the upper card member 4 is provided with a sample container/cup holding port 22 and an elogated aperture 8 into which a desiccant material may be placed. A wicking material 15, preferably nitro-cellulose, is placed between the upper and lower card members 4 and 6, respectively, and it extends underneath ports 10, 12, 14 and 16 with a portion of wicking material 15 being visible through ports 10, 12 and 14. The wicking material 15 is thus held in place between card members 4 and 6. At least a portion of wicking material 15 which is viewable through port 12 includes a binder specific for the analyte to be assayed and defines a test area. A first absorbent material, preferably in the form of an absorbent pad 18, is also placed between upper and lower card members 4 and 6, respectively, in such a manner that absorbent pad 18 is visible through port 16. Pad 18 is preferably in fluid or flow contact with wicking material 15. An indicator material or signal-producing substance may be applied to wicking material 15 viewable through port 10, to provide a signal indicating the completion of the assay or indicating a result. A second absorbent pad or "end" pad 26, shown in phantom lines on Fig. 1, is also placed between upper and lower card members 4 and 6 respectively, in such a manner that, albeit it may not be observable from the outside of the device 2, it is in fluid or flow contact with wicking material 15, preferably at the opposite end of wicking material 15 from first absorbent pad 18.

As particularly shown, the sample loading port 16 is closer to the tracer loading port 14 than the viewing windows, whereby the capillary flow path between ports 16 and 14 is shorter than the capillary flow path between ports 16 and 12.

In an assay, a sample to be assayed is applied through test port 16, a binder has been applied at part 12, and tracer has been applied through port 14. The sample flows along the wicking material 15 and contacts tracer at port 14. The sample then flows further along the wicking material 15 to test ports 12 and 10 respectively. As hereinabove indicated, the flow path of the wicking material is set up in a manner such that the sample contacts the tracer on the wicking material 15 prior to contact of the sample with the binder at port 12. The binding of tracer may then be determined through test port 12. The presence and/or amount of analyte present in the sample may be determined by the presence and/or amount of tracer as determined through port 12 of the device 2. In addition, if an indicator substance has been applied through test port 10, the completion of the assay may be determined.

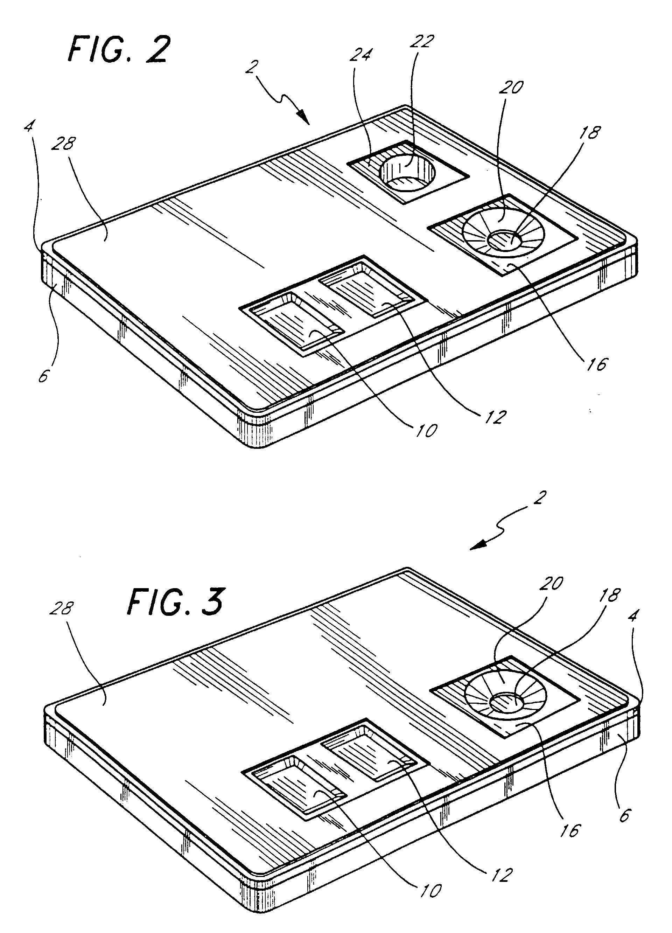

As shown in FIGS. 1 - 3, a thin web of opaque material 28 may also be applied over the top of the device 2 to cover various openings so that different assay formats can be provided using the same basic device 2 with different opaque webs 28. Thus, as illustrated in Figure 2, an assay can be provided using the same upper and lower card members 4, 6 shown in FIG. 1, except that the tracer loading port 14 and the aperture 8 have been covered by the opaque material 28. FIG. 3 corresponds to FIG. 2, except that the sample cup holding port 22 has also been covered by the opaque material 28.

In accordance with the preferred embodiment, as hereinabove described, by using an appropriate material as wicking material 15 (for example, nitro-cellulose) and a tracer which may include a visible particulate label (such as coloured latex), it is possible to determine binding of tracer through port 12 without destruction of the label.

Although the embodiment has been described with respect to the use of a tracer which includes a visible particulate label, it is to be understood that other detectable labels may be employed within the spirit and scope of the invention. For example, labels such as enzyme labels, chromogen labels, fluorescent and/or absorbing dyes may be used. In such cases, it may be necessary to add an additional substance in order to detect the label in port 12; for example, in the case of an enzyme label, a substrate may be added to produce detectible color in port 12. It is also possible to provide a second test port 10 to signal the end of the assay. In this manner, tracer which has not been bound in wicking material portion 12 may be determined. The presence and/or amount of tracer visible in port 12 may be employed to determine analyte alone and/or in conjunction with the presence and/or amount of analyte visible in port 10.

The invention can be better understood by way of the following examples which are representative of the preferred embodiments thereof, but which are not to be construed as limiting the scope of the invention.

### Example I

A reaction unit 2 was constructed using two plastic "card" members 4 and 6. The cards were made of polystyrene. Upper card 4 has a thickness of approximately 1mm. Lower card 6 has a thickness of approximately 1mm and has walls extending perpendicularly upward on all four sides, said walls joined at their corners, with said corners preferably being rounded, as illustrated in Fig. 1. When top card member 4 and bottom card member 6 are joined together, the unit has an apparent "thickness" of about 6mm. The top card member 4 has six apertures which include an elongated port 8, tracer loading port 14, sample loading port 16, sample cup holding port 22, wicking material visible under ports 10, 12, and 14, and test ports 10 and 12. Ports 10, 12 and 14 have dimensions of approximately 10mm X 10mm., while application port 16 has a diameter of about 7mm. Aperture 8 has a dimension of about 20mm X 50mm, while aperture 22 has a diameter of about 13mm.

Sample loading port 16 has shallow, sloping sides 20, which slope downward toward a circular aperture in which an absorbent material or pad 18, which is connected to the wicking material 15, is located. In a preferred embodiment, pad 18 is located underneath a portion of wicking material 15. A filtering device (not shown) may also be placed atop the sample loading port 16 to filter out particulates or other materials that may be in a sample to be applied. In another embodiment, additional absorbent material, in the form of pad 26, may be placed in fluid contact with wicking material 15 adjacent to port 10, for example, to absorb additional fluids once the sample has been wicked past port 10.

First absorbent pad 18 visible through port 16 receives the sample and releases it to the wicking material 15. Pad 18 may comprise, for example, a compact cellulose material such as D28, 17 Chr. (Whatman Manufacturing Inc., NJ), a more porous material such as GF/A or GF/D (Whatman Manufacturing Inc., NJ), or extra thick glass fiber filter (such as that available from Gelman Sciences, Michigan), depending upon the type of sample to be tested using the device. Compact absorbent pads are suitable for aqueous samples such as urine, and porous pads are preferred for viscous samples such as serum, plasma and blood. Essentially, the absorbent pad 18 retains the particulate or cellular fractions from the sample and allows the liquid portion to move towards the wicking material 15.

Absorbent end pad 26 preferably consists of materials that absorb and retain liquid and latex; this feature tends to restrict or prevent the reverse flow of latex, including colored latex. Without the end pad 26, the colored latex may begin to flow backwards approximately one hour after initiation of a test, and may appear in the "test complete" (port 10) and the "read result" (port 12) windows, resulting in a messy, uneven background. In addition, absorbent end pad 26 restricts or prevents the reverse flow of sample or sample components onto the wicking material.

Several absorbent pads were tested for their ability to prevent the reverse flow of coloured latex in a reaction unit similar to reaction unit 2. The absorbent materials which appeared to be better than the control pad alone (Whatman D28, Whatman Manufacturing Inc., NJ was used as the control pad) are as follows:
1. S&S 2727 (Schleicher and Schuell, W. Germany);
2. Multiform SG145 (Multiform Desiccants Inc., NY);
3. Pall Ultipor GF Plus 1.0µm (Pall BioSupport Corp., NY) on top of Whatman D28. (The test materials were a thin layer; thus, they were layered on top of control pad D28 to have proper contact with the wicking material.);
4. Dri Mop Liquid Absorber (Multiform Desiccants Inc., NY) on Whatman D28 or Dri Mop applied directly on the wicking material (15). (The test materials were powder gel; thus, they were sprinkled on top of control pad D28 to have proper contact with the wicking material.);
5. Ultra Pampers (Reg'd Trade Mark) Plus Diapers (Proctor & Gamble, OH);
6. Gelok 3000, 4000, 5500 and 6000 A/F single/double ply laminate with #1080 polymer, Gelok 5000/Scrim 3A, Gelok 5011-HS, Gelok 6000 Airlay/single ply laminate with IM-1000 polymer (all from Gelok International Corp., OH), on top of Whatman D28. (As before, the test materials were a thin layer; thus, they were layered on top of control pad D28 to have proper contact with the wicking material.)

Although the invention has been described in the context of particular embodiments, it is intended that the scope of coverage of the patent not be limited to those particular embodiments, but be determined by reference to the following claims.

## Claims

1. A method of constructing a plurality of immunoassay devices having a common multi-purpose housing (4, 6) but different finished appearances, comprising the steps of:
providing at least two identical housings (4, 6) each having an inside, an outside, and a top having a plurality of apertures (8, 10, 12, 14, 16, 24) communicating with the inside of the housing;
inserting a web (15) of wicking material inside each of said at least two identical housings extending between a first (16) second (12) and third (14) of said apertures, so that said wicking material is accessible through said first, second and third apertures and said third aperture is located between said first and second apertures;
applying a first reagent to said wicking material (15) through said second aperture (12), which first reagent is bound to said wicking material;
applying a second reagent to said wicking material through said third aperture (14) which second reagent is mobile when sample liquid comprising an analyte is added to said first aperture, and is adapted to co-operate with said analyte in said sample and said first reagent to create a signal indicative of the results of said assay, which signal can be read through said third window; and thereafter,
applying a first thin web (28) of opaque material over the top of a first one of said at least two identical housings such that of the three apertures, only said first and second apertures are uncovered, to form a first immunoassay device; and
applying a second thin web (28) of opaque material over the top of a second one of said at least two identical housings such that said first and second apertures and at least one additional aperture are uncovered to form a second immunoassay device.

2. The method of Claim 1, further comprising the step of inserting an absorbent material (18) into the first one of said at least two identical housings before applying the top of the housing.

3. The method of Claim 1, wherein the top of said first one of said at lest two identical housings has an opening (8) therethrough for receiving a desiccant, further comprising the steps of:
inserting a desiccant into said housing through said opening before applying said first thin web (28) and applying said first thin web over said opening.

4. The method of Claim 1, wherein said wicking material (15) also extends to a fourth said aperture (10), said fourth aperture adapted to permit viewing of a signal on said wicking material indicative of completion of said assay.

5. The method of Claim 4, further comprising an absorbent material (26) in fluid flow contact with said wicking material (15) adjacent to said fourth aperture (10).

6. The method of Claim 1, wherein the top of said housing further has a well (22) formed therein for holding a container for liquid used in said assay.

7. The method of Claim 1, wherein said thin webs comprise paper.

## Patentansprüche

1. Verfahren zur Herstellung einer Vielzahl von Immunoassay - Vorrichtungen, welche ein gemeinsames Mehrzweckgehäuse (4, 6), aber unterschiedliche Enderscheinungen aufweisen, mit den folgenden Schritten:
Zur Verfügungstellen von mindestens zwei identischen Gehäusen (4, 6), wobei jedes Gehäuse eine Innenseite, eine Außenseite sowie einen mit mehreren mit der Innenseite des Gehäuses kommunizierenden Öffnungen (8, 10, 12, 14, 16, 24) versehenen Deckel aufweist;
Einführen eines aus einem Dochtmaterial bestehenden Webstoffes (15) in jedes der mindestens zwei identischen Gehäuse, wobei sich der Webstoff zwischen einer ersten (16), einer zweiten (12) und einer dritten (14) Öffnung erstreckt, derart daß das Dochtmaterial durch die erste, zweite und die dritte Öffnung, welche zwischen der ersten und der zweiten Öffnung angeordnet ist, erreichbar ist;
Auftragen eines ersten Reagenzes, welches an dieses Dolchmaterial gebunden wird, durch die zweite Öffnung (12) auf das Dochtmaterial (15);
Auftragen eines zweiten Reagenzes auf das Dochtmaterial durch die dritte Öffnung (14), wobei das zweite Reagenz dünnflüssig ist, wenn eine Probeflüssigkeit, die einen Analyten enthält, der ersten Öffnung hinzugefügt wird, sowie mit dem Analyten in der Probe und mit dem ersten Reagenz zusammenwirken kann, um ein Indikationssignal der Resultate der Untersuchung zu generieren, welches durch das dritte Fenster abgelesen werden kann; und danach,
Anbringen eines ersten dünnen Webstoffes (28) aus undurchsichtigem Material über den Deckel eines ersten der mindestens zwei identischen Gehäuse, so daß nur die erste und die dritte der drei Öffnungen unbedeckt sind, um eine erste Immunoassay - Vorrichtung zu formen; und
Anbringen eines zweiten dünnen Webstoffes (28) aus undurchsichtigem Material über den Deckel eines zweiten der mindestens zwei identischen Gehäuse, so daß die erste und die zweite Öffnung und mindestens eine zusätzliche Öffnung unbedeckt sind, um eine zweite Immunoassay - Vorrichtung zu formen.

2. Verfahren nach Anspruch 1, bei dem in einem weiteren Schritt vor dem Anbringen des Gehäusedeckels ein absorbierendes Material (18) in das erste der mindestens zwei identischen Gehäuse eingeführt wird.

3. Verfahren nach Anspruch 1, bei dem der Deckel des ersten der mindestens zwei identischen Gehäuse eine durchgehende Öffnung (8) aufweist, um ein Sikkativ aufzunehmen und bei dem zusätzlich die Schritte enthalten sind:
Einführen eines Sikkativs in das Gehäuse durch diese Öffnung vor dem Anbringen des ersten dünnen Webstoffes (28) sowie dem Anbringen des ersten dünnen Webstoffes über diese Öffnung.

4. Verfahren nach Anspruch 1, bei dem sich das Dochtmaterial (15) auch zu einer vierten Öffnung (10) erstreckt, welche das Beobachten eines die Vollendung der Untersuchung anzeigenden Signals auf diesem Dochtmaterial ermöglicht.

5. Verfahren nach Anspruch 4, bei dem zusätzlich ein an die vierte Öffnung (10) angrenzendes absorbierendes Material (26) in Fluid- Flusskontakt mit dem Dochtmaterial (15) vorgesehen ist.

6. Verfahren nach Anspruch 1, bei dem der Deckel des Gehäuses zusätzlich einen darin geformten Behälter (22) zum Halten eines Behälters für die in der Untersuchung verwendete Flüssigkeit aufweist.

7. Verfahren nach Anspruch 1, bei dem die dünnen Webstoffe Papier enthalten.

## Revendications

1. Méthode pour construire une pluralité de dispositifs d'essais immunologiques ayant un logement commun universel (4-6) mais avec deux finitions différentes, comprenant les étapes de :
la fourniture d'au moins deux logements identiques (4, 6) ayant chacun une partie interne, une partie externe, et une partie supérieure, comportant une pluralité d'ouvertures (8, 10, 12, 14, 16, 24) communiquant avec l'intérieur du logement;
l'insertion d'un film (15) en matériau à effet de mèche à l'intérieur de chacun desdits au moins deux logements identiques, s'étendant entre la première (16) la deuxième (12) et la troisième (14) desdites ouvertures, de sorte que le dit matériau à effet de mèche est accessible à travers lesdites première, deuxième et troisième ouvertures, et ladite troisième ouverture est placée entre lesdites première et deuxième ouvertures :
l'application d'un premier réactif sur ledit matériau (15) à effet de mèche à travers ladite deuxième ouverture (12), ledit premier réactif étant lié audit matériau à effet de mèche ;
l'application d'un second réactif sur ledit matériau à effet de mèche, à travers ladite troisième ouverture (14), le second réactif étant mobile lorsque le liquide de l'échantillon comprenant un "analyte" est ajouté à ladite première ouverture, et est adapté pour coopérer avec ledit "analyte" dans ledit échantillon et ledit premier réactif pour créer un signal indicateur des résultats dudit essai, lequel signal peut être lu à travers ladite troisième fenêtre ; et ensuite,
l'application d'un premier film fin (28) de matériau opaque sur la partie supérieure du premier desdits au moins deux logements identiques de sorte que, sur les trois ouvertures, seules lesdites première et deuxième ouvertures sont découvertes, pour former un premier dispositif d'essai immunologique ; et
l'application d'un second film fin (28) de matériau opaque sur la partie supérieure du second desdits au moins deux logements identiques de sorte que lesdites première et deuxièmes ouvertures et au moins une ouverture supplémentaire sont découvertes pour former un deuxième dispositif d'essai immunologique.

2. Méthode selon la revendication 1, comprenant en outre l'opération d'insertion d'un matériau absorbant (18) dans le premier desdits au moins deux logements identiques avant l'application de la partie supérieure du logement.

3. Méthode selon la revendication 1, dans laquelle la partie supérieure dudit premier desdits au moins deux logements identiques a une ouverture (8) traversante pour recevoir un produit déshydratant, comprenant en outre les étapes de :
l'insertion d'un déshydratant dans ledit logement à travers ladite ouverture, avant l'application dudit premier film fin (28) et l'application duddit film fin sur ladite ouverture.

4. Méthode selon la revendication 1, dans laquelle ledit matériau (15) à effet de mèche s'étend également vers ladite quatrième ouverture (10), ladite quatrième ouverture étant adaptée pour permettre de voir un signal sur ledit matériau à effet de mèche, indiquant l'achèvement dudit essai.

5. Méthode selon la revendication 4, comprenant en outre un matériau absorbant (26) en contact avec le débit de fluide dudit matériau (15) à effet de mèche adjacent à ladite quatrième ouverture (10).

6. Méthode selon la revendication 1, dans laquelle la partie supérieure dudit logement comporte en outre un puits (22) formé à l'intérieur pour maintenir un conteneur pour le liquide utilisé pendant ledit essai.

7. Méthode selon la revendication 1, dans laquelle lesdits films fins comprennent du papier.
